# EUROPEAN PATENT SPECIFICATION

(11) **EP 1 856 035 B1**
(45) Date of publication and mention of the grant of the patent: **10.12.2008**
(21) Application number: 06709867.3
(22) Date of filing: 22.02.2006
(51) Int. Cl.: C07C 259/14, C07C 259/18, C07C 271/22, C07D 205/04, C07D 209/18

(54) **PROCESS FOR PREPARAING PROTECTED AMIIDINES**
VERFAHREN ZUR HERSTELLUNG GESCHÜTZTER AMIDINE
PROCEDE DE PREPARATION D'AMIDINES PROTEGEES

(30) Priority: 23.02.2005 GB 0503672
(43) Date of publication of application: 21.11.2007
(73) Proprietor: AstraZeneca AB, 151 85 Södertälje (SE)
(72) Inventor: AL-SAFFAR, Firas, S-151 85 Södertälje (SE); BERLIN, Stefan, S-151 85 Södertälje (SE); MUSIL, Tibor, S-151 85 Södertälje (SE); SIVADASAN, Sivaprasad, S-151 85 Södertälje (SE)
(86) International application number: PCT/GB2006/000635
(87) International publication number: WO 2006/090153

(56) References cited:
- RACHID BAATI ET AL: "An Improved Method for the Preparation of Amidines via Thiophenylimidic Esters" SYNTHESIS, no. 6, 1999, pages 927-929, XP002384285
- NAVAMONEY ARULSAMY AND D. SCOTT BOHLE: "Nucleophilic Addition of Hydroxylamine, Methoxylamine, and Hydrazine to Malononitrileoxime" J. ORG. CHEM., vol. 65, 2000, pages 1139-1143, XP002384286
- KOSHIO H ET AL: "Orally active factor Xa inhibitor: synthesis and biological activity of masked amidines as prodrugs of novel 1,4-diazepane derivatives" BIOORGANIC & MEDICINAL CHEMISTRY, ELSEVIER SCIENCE LTD, GB, vol. 12, no. 20, 15 October 2004 (2004-10-15), pages 5415-5426, XP004573362 ISSN: 0968-0896

## Description

The invention concerns a new process for the preparation of certain protected amidines, such as alkoxyamidines, from nitriles. The method may be used, for example, with benzonitriles bearing electron donating or electron withdrawing groups, and is useful, for example, in the preparation of intermediates, for example for use in the manufacture of thrombin inhibitors. The process shows improvement and advantage compared to alternative preparation methods for such compounds.

The amidine moiety is important for the therapeutic activity of new competitive inhibitors of trypsin-like proteases (such as thrombin). To improve the oral bioavailability of amidine direct thrombin inhibitors such as melagatran, the amidine group may be protected, for example as a hydroxyamidine (such as in ximelagatran) or as an alkoxyamidine group. Once administered, the hydroxy- or alkoxy-amidine is reduced "in vivo" to an amidine group.

Examples of compounds, other than melagatran, that are, or are metabolised to compounds which are, competitive inhibitors of trypsin-like proteases (such as thrombin) are described in International Patent Application No. PCT/SE01/02657 (WO 02/44145). This application discloses, for example, compounds of formula IA, 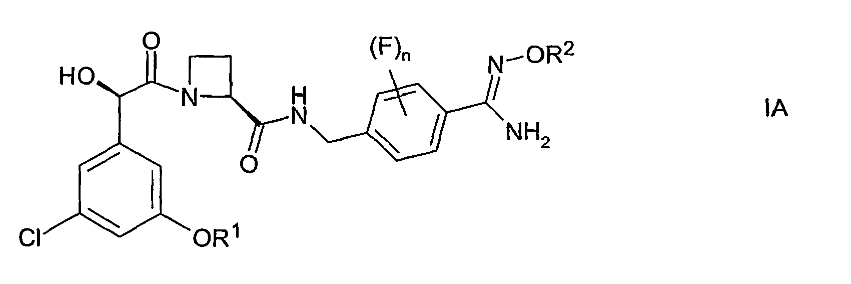 wherein
R¹ represents C₁₋₂ alkyl substituted by one or more fluoro substituents;
R² represents C₁₋₂ alkyl; and
n represents 0, 1 or 2; and also the following two compounds :
(a) Ph(3-Cl)(5-OCHF₂)-(*R*)CH(OH)C(O)-(*S*)Aze-Pab(OMe): 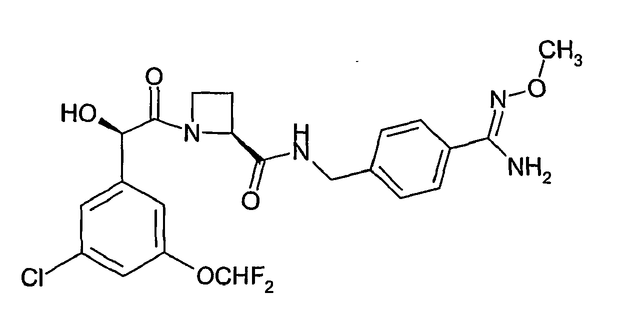 which compound is referred to hereinafter as Compound A;
(b) Ph(3-Cl)(5-OCHF₂)-(*R*)CH(OH)C(O)-(*S*)Aze-Pab(2,6-diF)(OMe): 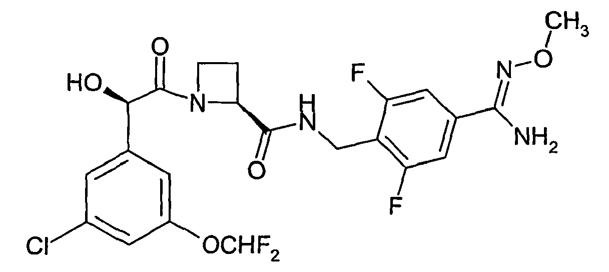 which compound is referred to hereinafter as Compound B.

Alkoxyamidines, such as methoxyamidine, are generally prepared by a multi-step synthesis where a nitrile is first converted to an amidine or hydroxyamidine and then converted to methoxyamidine, for example using methoxylamine hydrochloride. However, this chemistry is not particularly satisfactory (for example, in yield or reaction time) for most substrates. There is thus a need for simpler and more efficient methods of preparing protected amidines, such as alkoxyamidines. The method of the present invention offers such advantages by performing this transformation in one single step from nitriles. The process is also particularly useful for preparing protected amidines, such as alkoxyamidines, which are acid sensitive, or have acid-sensitive groups.

The preparation of amidines is described, for example, in WO 98/09950 in which a nitrile is reacted with ammonia, an alkylamine or hydrazine in the presence of a thiocarboxylic acid.

The preparation of hydroxyamidines from nitriles can be accomplished using hydroxylamine. Conversion of such hydroxyamidines to alkoxyamidines requires further alkylation chemistry which is not particularly suitable for large scale manufacturing purposes.

Synthesis of alkoxyamidines, such as methoxyamidine, from amidines can be demonstrated, for example using methoxylamine, but such chemistry first requires formation of the amidine moiety. The preparation of protected (for example, alkoxy)amidines directly from nitriles is not known.

Additionally, optionally substituted benzonitriles generally need to be activated by strong acids, such as hydrochloric acid, or Lewis acids, such as trimethyl-aluminium, to react at the nitrile.

The process of the present invention has overcome these problems, and offers the opportunity to prepare protected amidines directly from nitriles in good yield, and without requiring strong acids, such as hydrochloric acid, or Lewis acids, such as trimethyl-aluminium. As will be seen, the only acid that may be considered to be present is a thiocarboxylic acid, but the acidity of this acid is not required to perform the reaction (the reaction has been shown to occur to approximately the same extent with ethyl mercaptoacetate - see Example 1).

The process of the invention is particularly applicable to the preparation of compounds such as those described in WO 02/44145, i.e. compounds of formula IB, 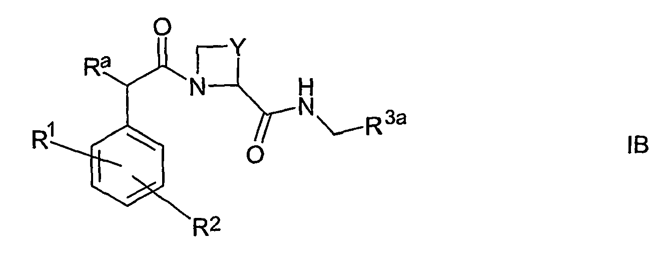 wherein R^{3a} represents a structural fragment of formula I(i) or I(ii): 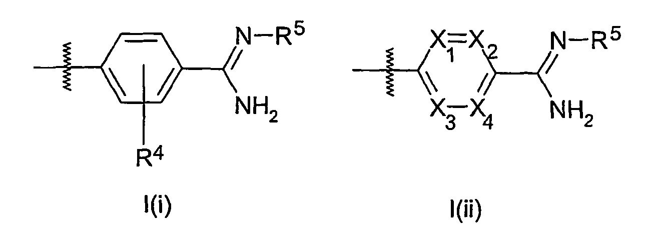 wherein R⁵ is -OR⁶ and R⁶ represents C₁₋₁₀ alkyl, C₁₋₃ alkylaryl or C₁₋₃ alkyloxyaryl (the alkyl parts of which latter two groups are optionally interrupted by one or more oxygen atoms, and the aryl parts of which latter two groups are optionally substituted by one or more substituents selected from halo, phenyl, methyl or methoxy, which latter three groups are also optionally substituted by one or more halo substituents); and
R^{a} represents -OH or -CH₂OH;
R¹ represents one or more optional halo substituents;
R² represents one or two C₁₋₃ alkoxy substituents, the alkyl parts of which substituents are themselves substituted by one or more fluoro substituents;
Y represents -CH₂- or -(CH₂)₂-;
R⁴ represents H or one or more fluoro substituents; and
one or two of X₁, X₂, X₃ and X₄ represent -N- and the others represent -CH-.

Accordingly, the present invention provides a process for preparing a protected amidine group of formula (I) 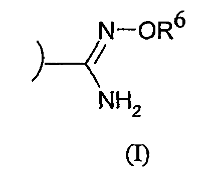 wherein R⁶ represents C₁₋₁₀ alkyl (optionally substituted by one or more substituents independently selected from halo, C₁₋₄ alkoxy, nitro, C₁₋₄ alkylamine and di-(C₁₋₄ alkyl)amine), aryl, C₁₋₃ alkylaryl or C₁₋₃ alkyloxyaryl (the alkyl parts of which latter two groups are optionally interrupted by one or more oxygen atoms, and the aryl parts of which latter two groups are optionally substituted by one or more substituents selected from halo, phenyl, methyl or methoxy, which latter three groups are also optionally substituted by one or more halo substituents)
which comprises
reacting a nitrile containing compound with an oxyamine of formula (II)

R⁶ONH₂ (II)

wherein R⁶ is as defined above for (I);
in the presence of a thio-keto activating agent of formula (III)

HS-R_{y}-C(O)-Z (III)

wherein Z is -(1-4C)alkyl, -OH, -O-(1-4C)alkyl, -SH, -S(1-4C)alkyl, -NH₂, -NH(1-4C)alkyl or -N[(1-4C)alkyl]₂ ;
R_{y} is (1-2C)alkyl, which is optionally substituted by up to three substituents independently selected from (1-4C)alkyl, halo, amino and acetylamino; or
Z and Ry are linked so as to form a 5- or 6-membered ring of formula (IV) 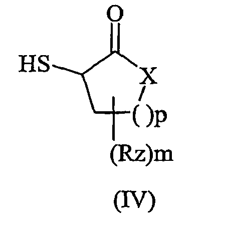 wherein
X is -CH₂-, -O-, NH- or -N(1-4C)alkyl; p is 1 or 2; m is 1 or 2 and
Rz is independently selected from H, (1-4C)alkyl, halo and amino.

By protected amidine we mean an amidine moiety of formula (I) 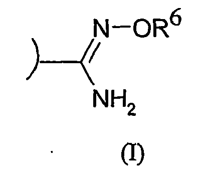 wherein R⁶ represents C₁₋₁₀ alkyl (optionally substituted by one or more substituents independently selected from halo, C₁₋₄ alkoxy, nitro, C₁₋₄ alkylamine and di-(C₁₋₄ alkyl)amine), aryl, C₁₋₃ alkylaryl or C₁₋₃ alkyloxyaryl (the alkyl parts of which latter two groups are optionally interrupted by one or more oxygen atoms, and the aryl parts of which latter two groups are optionally substituted by one or more substituents selected from halo, phenyl, methyl or methoxy, which latter three groups are also optionally substituted by one or more halo substituents).

Accordingly, the oxyamine is of formula (II)

R⁶ONH₂ (II)

wherein R⁶ is as defined above for (I).

Alkyloxyaryl groups that R⁶ may represent, comprise an alkyl and an aryl group linked by way of an oxygen atom. Alkylaryl (for example benzyl) and alkyloxyaryl groups are linked to the rest of the molecule *via* the alkyl part of those groups, which alkyl parts may (if there is a sufficient number (i.e. three) of carbon atoms) be branched-chain. Aryl, and the aryl parts of alkylaryl and alkyloxyaryl groups which R⁶ may represent, or be substituted by, include carbocyclic and heterocyclic aromatic groups, such as phenyl, naphthyl, pyridinyl, oxazolyl, isoxazolyl, thiadiazolyl, indolyl and benzofuranyl and the like.

Alkyl groups which R⁶ may represent may be straight-chain or, when there is a sufficient number (i.e. a minimum of three) of carbon atoms, be branched-chain and/or cyclic. Further, when there is a sufficient number (i.e. a minimum of four) of carbon atoms, such alkyl groups may also be part cyclic/acyclic. Such alkyl groups may also be saturated or, when there is a sufficient number (i.e. a minimum of two) of carbon atoms, be unsaturated.

Halo groups with which R⁶ may be substituted include fluoro, chloro, bromo and iodo, especially F or Cl.

Particular values for R⁶ are C₁₋₄ alkyl, especially methyl and ethyl, and phenyl.

The nitrile containing compound includes any molecule containing a nitrile group which forms all, or part of, a final molecule in which a protected amidine group is to be introduced. Suitable nitrile containing compounds include aromatic nitriles (such as optionally substituted cyano-benzene compounds), heteroaromatic nitriles, heterocyclic nitriles, alkyl nitriles (such as optionally substituted (1-4C)alkyl chains, such as optionally substituted benzyl-nitriles) and cyclo-alkyl nitriles (such as optionally substituted (3-5C)cyclo-alkyl rings).

Heteroaromatic nitriles include aromatic ring systems containing 1-3 heteroatoms independently selected from N, O and S. Heterocyclic nitriles include non-aromatic rings containing 1-3 heteroatoms independently selected from N, O and S.

Any ring in the nitrile containing compound may be optionally substituted by other group/s forming part of a final molecule to be prepared, or for example, on an available carbon atom by up to three (preferably one) substituent/s independently selected from halo, (1-4C)alkyl and (1-4C)alkoxy.

The thio-keto activating agent is of formula (III)

HS-R_{y}-C(O)-Z (III)

wherein Z is -(1-4C)alkyl, -OH, -O-(1-4C)alkyl, -SH, -S(1-4C)alkyl, -NH₂, -NH(1-4C)alkyl or -N[(1-4C)alkyl]₂ ;
R_{y} is (1-2C)alkyl, which is optionally substituted by up to three substituents independently selected from (1-4C)alkyl, halo, amino and acetylamino; or
Z and Ry are linked so as to form a 5- or 6-membered ring of formula (IV) 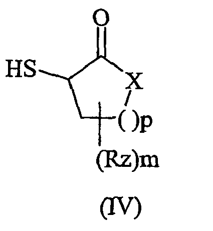 wherein
X is -CH₂-, -O-, NH- or -N(1-4C)alkyl; p is 1 or 2; m is 1 or 2 and
Rz is independently selected from H, (1-4C)alkyl, halo and amino.

The thio-keto activating agent of formula (III) or (IV) is believed to activate the nitrile group, and hydrogen bonding between the keto and thiol functionality may be important in this respect. The thio-keto activating agent is generally used in quantitative amounts (i.e. approximately equi-molar with the nitrile compound), although lower (e.g. 1 : 0.5) and higher (e.g. 1 : 1.5) molar ratios of nitrile compound : thio-keto activating agent may be employed provided the reaction proceeds satisfactorily.

Thio-keto activating agents of formula (III) may include (alpha)-thiocarboxyclic acids, equivalent esters (for example the methyl or ethyl ester) and amino acids such as cysteine (zwitterionic) or N-acetylcysteine. A preferred thio-keto activating agent is of formula (III) wherein Z is-OH or -O-(1-4C)alkyl, particularly mercapto acetic acid.

Certain thio-keto activating agents of formula (III) or (IV) may also be employed in the form of an appropriate salt.

Certain reagents and products disclosed in this application may exhibit tautomerism. All tautomeric forms and mixtures thereof are included within the scope of the invention.

The process of the invention is peformed in any suitable solvent, for example an alcohol (such as methanol or ethanol or n-butanol), an acetate (such as ethyl acetate), water or a mixture of such solvents. Other possible solvents are aromatic solvents, chlorinated solvents and oxygenated solvents, such as ethers.

The process of the invention is peformed at any suitable temperature, for example at the reflux temperature of the reaction mixture.

A metal chelating agent (for example EDTA) may be added to chelate metal ion impurities (such as iron ions) which may lead to formation of impurities/by-products during the process of the invention.

A base, such as NaOH, triethylamine or N-methylmorpholine, may be used, for example to deprotect a salt form of an oxyamine during the process of the invention.

It should be noted that in certain nitrile containing compounds and/or in certain thio-keto activating agents there may be groups (for example, amino groups) which may require protection during the process of the invention. Such groups may be deprotected if appropriate after formation of the protected amidine functionality. Thus, for example, amine groups may be optionally protected using such standard protecting groups as Boc, mesylate, tosylate or benzyl. Such groups can be removed using standard techniques.

Particular illustrations of the process of the invention are shown by the following scheme 1 for benzonitriles (other substrates and reagents may be used) :- 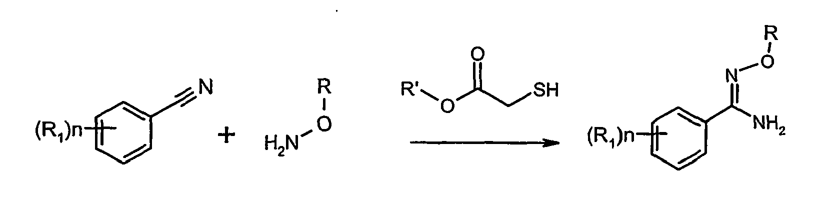 R = optionally substituted (1-10C)alkyl, phenyl or benzyl;
R' = H or (1-10C)alkyl;
n=0,1,2or3;
R₁ = H, halogen, optionally substituted (1-4C)alkyl, aromatic group, or any group (or combination of groups) which form all, or part of, a compound in which a protected amidine group is (to be) present.
R and R' may be the same or different; if n is 2 or 3, each R₁ may be the same or different.

### Scheme 1

Examples of R₁ groups in Scheme 1 include those of formula II below :- 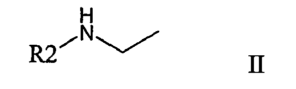 wherein R2 is, for example, H, Boc or a group of the formula III :- 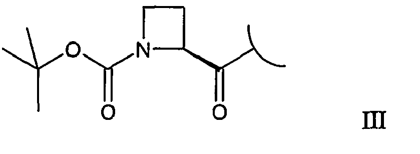 When n is 1, preferred reactions are those which produce the following products (wherein R is methyl (Me in named compound) or ethyl) suitable for use in preparing Compound A:-
(a) Pab(OMe) 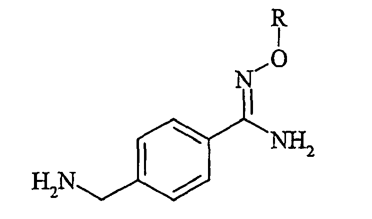
(b) Boc-Pab(OMe) 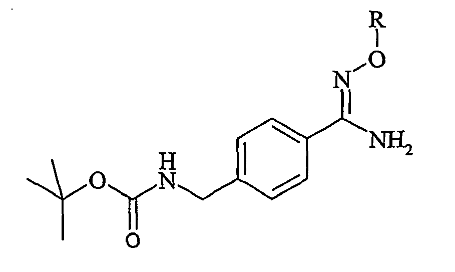
(c) Aze-Pab(OMe) 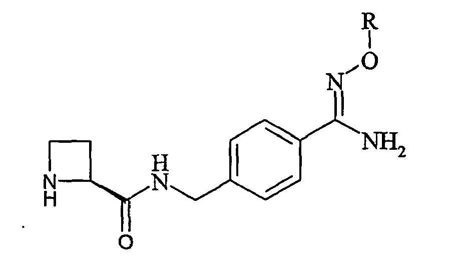
(d) Boc-Aze-Pab(OMe) 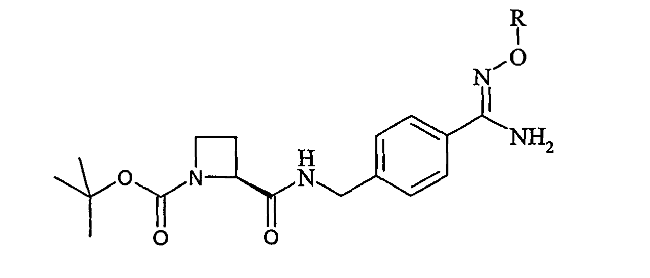
When n is 3, preferred reactions are those which produce the following products (wherein R is methyl (Me in named compound) or ethyl), suitable for use in preparing Compound B:-
(a) (2,6-diF)Pab(OMe) 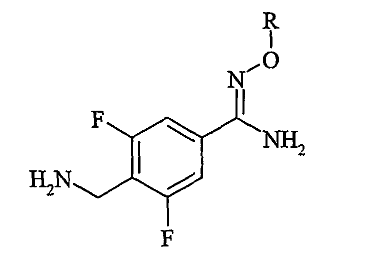
(b) Boc-(2,6-diF)Pab(OMe) 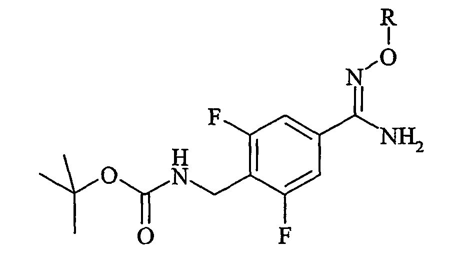
(c) Aze-(2,6-diF)Pab(OMe) 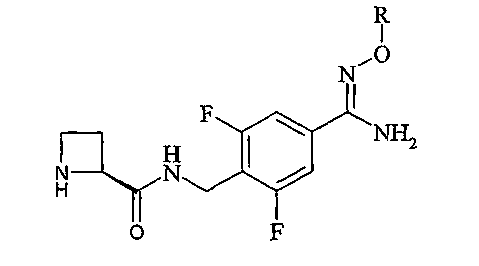
(d) Boc-Aze-(2,6-diF)Pab(OMe) 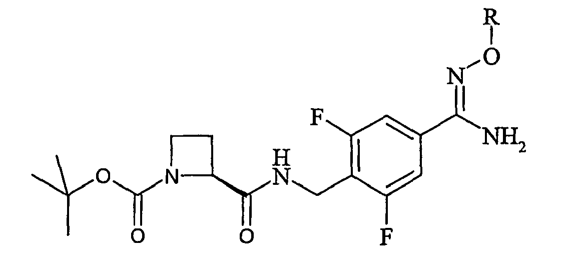 To prepare compounds A and B, Aze-Pab(OMe) and Aze-(2,6-diF)Pab(OMe) respectively may be coupled (using standard conditions) to 3-chloro-5-difluoromethoxy mandelic acid (which may itself be prepared, for example according to the Scheme A below, in which the skilled chemist will be able to perform the relevant transformations. The desired chiral mandelic acid may be obtained, for example, by resolution from a chiral/racemic mixture).

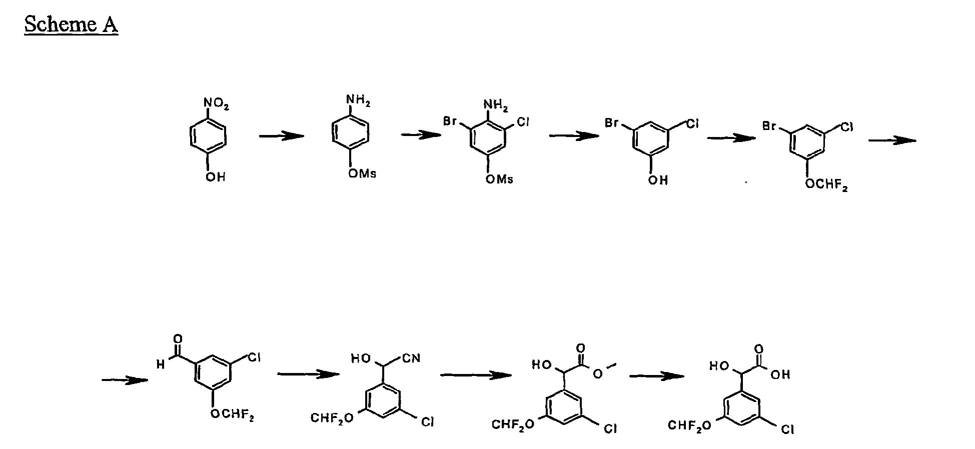

### EXAMPLES

The invention will now be illustrated by the following non-limiting Examples in which ...
Aze = (*S*)-azetidine-2-carboxylate (unless otherwise specified)
Boc = *tert*-butoxycarbonyl
CBA = *para*-cyano-benzylamine
Pab = *para*-amidinobenzylamino
EDC = 1-(3-dimethylaminopropyl)-3-ethylcarbodiimide hydrochloride
TBTU = [*N*,*N*,*N*',*N*'-tetramethyl-*O*-(benzotriazol-1-yl)uronium tetrafluoroborate]
TCTU = N-[(1H-6-chlorbenztriazol-1-yl) (dimethylamino)methylene]-N-methylmethanaminium tetrafluoroborate N-oxide
HBTU = N-[(1H-benztriazol-1-yl) (dimethylamino)methylene]-N-methylmethanaminium hexafluorophosphate N-oxide
HCTU = N-[(1H-6-chlorbenztriazol-1-yl) (dimethylamino)methylene]-N-methylmethanaminium hexafluorophosphate N-oxide
PyBOP = Benzotriazol-1-yl-N-oxy-tris(pyrrolidino)phosphonium hexafluorophosphate

### Example 1

Methoxylamine hydrochloride (0.88 g, 10.4 mmol) was mixed with triethylamine (1.45 ml, 10.4 mmol; or a slight excess of triethylamine against the methoxylamine hydrochloride may be used) in ethanol (20 ml). 3,5-difluorobenzonitrile (0.5 ml, 4.9 mmol) and mercaptoacetic acid (0.34 ml, 4.9 mmol) was added and the mixture was heated to reflux and left so for about 6-12 hours. The reaction was cooled and concentrated under reduced pressure.

Other N'-methoxyarylamidines were prepared by analogy with the description above for the synthesis of N'-methoxybenzamidines, and are presented in Table 1.

### General method for work-up for all reactions in Example 1:

The residue was dissolved in ethyl acetate and washed with 5%w/w aqueous Na₂CO₃. The organic phase was dried over MgSO₄, evaporated and applied to a silica column. The product was extracted out with a mixture of dichloromethane and methanol, ethanol or ethyl acetate. The crude N'-methoxyarylamidines were obtained as an oil or solid residue after evaporation of the solvents.

Yields are presented as a conversion in terms of GC area% of the expected product in the crude reaction mixture, with the exception of Boc-Aze-(2,6-diF)CBA and Boc-Aze-CBA, where HPLC was used. Structures were confirmed on isolated materials obtained by the general purification method described in Example 1, either by GC- or LC-MS and/or NMR.

**Table 1 N'-methoxyarylamidines**

| **Nitrile** | **Thio-keto activating agent** | **Conversion** |
|---|---|---|
| Benzonitrile | Mercaptoacetic acid | 74 % |
| | Ethyl-2-mercaptoacetate | 39 % |
| 4-Chlorobenzonitrile | Mercaptoacetic acid | 82 % |
| 3,5-Difluorobenzonitrile | | 97 % |
| 4-Methoxybenzonitrile | | 54 % |
| Boc-Aze-(2,6-diF)CBA | | 89 % |
| Boc-Aze-CBA | | 43 % |

Using the process of the invention, the transformation of Boc-CBA to Boc-Pab(OMe) was performed in ethanol (Example 2) and the transformation of Boc-Aze-CBA to Boc-Aze-Pab(OMe) was performed with either methanol or ethanol as solvent (Example 3). With both substrates, good conversions were obtained using mercaptoacetic acid catalyst.

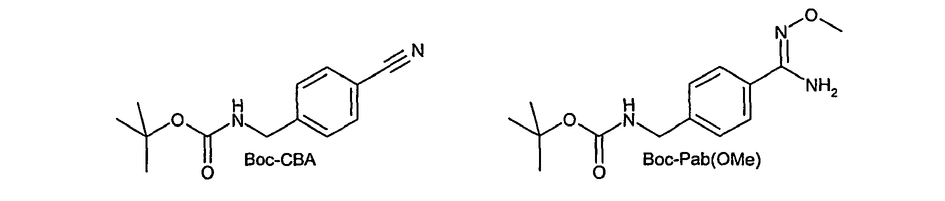

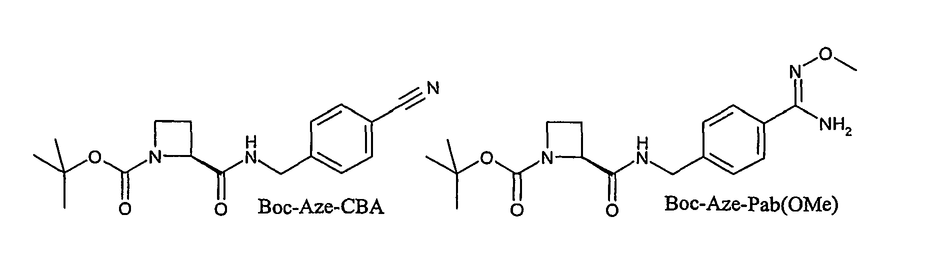

### Example 2

Pab(OMe) was prepared from CBA.HCl as follows:-

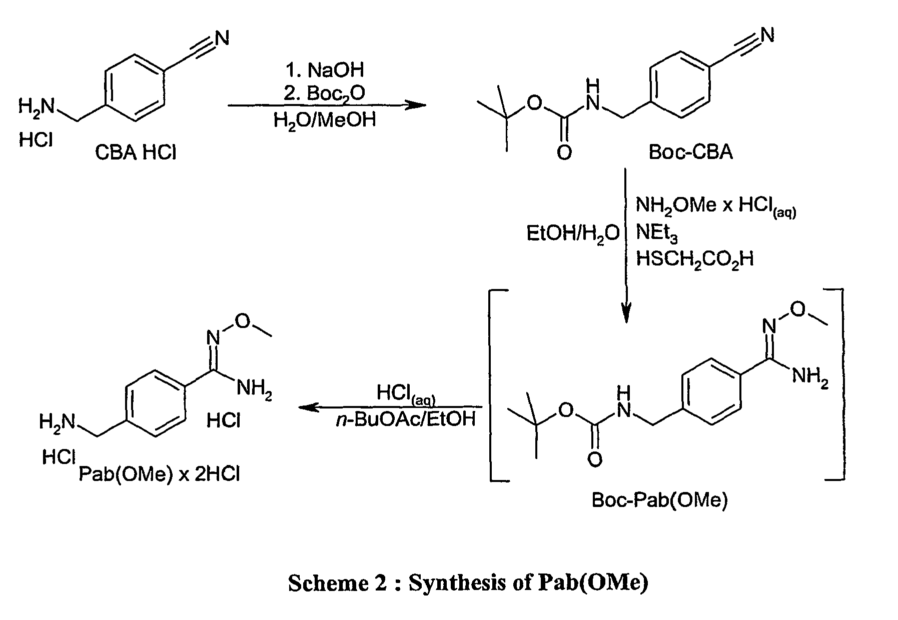

### Step 1: tert-Butoxycarbonylation of CBA HCl salt (to give Boc-CBA)

4-Cyanobenzylamine hydrochloride salt (CBA HCl) (200 g, 1.19 mol) was dissolved in 1.25 L of distilled deionised water (6.25 volumes) in a 5 L reactor with a mantle temperature of 25 °C. At 25 °C, the solution was clear within 15 minutes. To the clear solution was added a solution of 52 g (1.1 eq.) of NaOH in 400 mL of water. The solution was added at a rate of 10-15 mL/min. A small temperature increase (~3 °C) was noted. A large amount of precipitate (the free amine) appeared during the addition. After the addition, the pH of the mixture was >12.

A solution of Boc₂O (285 g, 1.30 mol, 1.1 eq.) in MeOH (200 mL) was added drop-wise to the white suspension at an average rate of∼10 mL/min. Carbon dioxide evolution and a small temperature increase (5 °C over 30 min) was noted during the addition. As the addition continued, the consistency of the slurry/suspension changed as the insoluble amine was converted to the insoluble Boc-protected amine.

The reaction was followed by taking samples of the supernatant in the reactor and comparing the peak area of the starting material with a calibration curve of the starting material (HPLC column: Symmetry Shield RP8, 3.5 µm, 50 mm; 205 and 230 nm - see Example 3 for more details). A 98% conversion was obtained 6 hours after the addition of Boc₂O was complete. The reaction was left overnight to obtain a 99% conversion. The slurry was filtered and the solid washed with 2 x 500 mL water. The material was dried under vacuum at 40 °C to give Boc-CBA (265g, 97% yield, 98.7% purity (HPLC by analogy to Example 3, no Boc₂O detected). Step 2: Methoxyamidination of Boc-CBA (to give Boc-Pab(OMe))

A 5 L reactor was flushed with N₂ and EtOH (1.75 L, 7 vol) was added. The N₂ was allowed to flow through the reactor while the following additions were made. Boc-CBA (250g, 1.08 mol) was added to the EtOH and the temperature was raised to 55°C. After almost complete dissolution, triethylamine (525 mL, 3.5 eq.) was added, and then a 30% w/w solution of NH₂OMe hydrochloride in water (599 g, 2 eq.) was added drop-wise at a rate of∼20 mL/min. (the NH₂OMe salt solution used had a 5% wt. excess of HCl in it so 3 eq. of base was needed to neutralize all the HCl in the reagent). A small exotherm was noted, and during the addition, the solution cleared.

α-Mercaptoacetic acid (77mL, 1 eq.) was added to produce a slightly opaque mixture of total volume ~3 L. The mantle was heated carefully to 90 °C., and when reflux (internal temp = 78 °C) was obtained, the N₂ was turned off and the reaction stirred overnight.

After 21 hours of stirring at reflux, the reaction was at 96% conversion (HPLC column: Symmetry Shield RP8, 3.5 µm, 50 mm; 230 nm - see Example 3 for more details). The reaction mixture was then cooled to 40 °C and acetone (160 mL, 2 eq.) added over one minute. The mixture was allowed to stand for 1 hour in order to quench any remaining NH₂OMe. The mixture was then poured into round-bottomed flasks and the solvent removed by rotary evaporation at 40 °C under vacuum. The remaining residues were dissolved in 3 L of a 2:1 mixture of nBuOAc: H₂O and placed in a reactor at 25 °C (total volume = 4.5 L) and stirred for 10 minutes for efficient mixing of the layers.

After the phases separated on standing, the aqueous layer (2 L, pH 6) was removed. The organic layer was washed with 1 L of water and the aqueous layer removed (pH=4). This was followed by a wash with a solution of K₂CO₃ (297 g, 2 eq.) in 1 L of water (this helps remove the mercaptoacetic acid). The basic water layer was removed and a final wash with 1 L of water was performed. After removal of the aqueous layer, the organic layer was removed from the reactor and stored overnight (total vol ∼2.3 L).

The organic layer was azeotropically dried by removal of approximately half of the nBuOAc (925 mL) on a rotary evaporator. The resulting mixture was placed in a reactor (mantle temp. = 30°C) and 425 mL of nBuOAc was added (total volume of nBuOAc = 2 L - 0.925 + 0.425 = 1.5 L = 6 vol). This was followed by addition of 440 mL (1.75 vol.) of EtOH. The mixture was heated to 30 °C to obtain a solution of Boc-Pab(OMe).

The above reaction can be repeated using n-butanol as solvent in place of ethanol.

The Boc-CBA obtained by the step 1 reaction above can also be used without isolation or purification in the step 2 methoxyamidation reaction.

### Step 3: Deprotection of tert-butoxycarbonyl to give dihydrochloride salt of Pab(OMe)

The above nBuOAc/EtOH solution of Boc-Pab(OMe) was stirred and concentrated (12 M) aqueous HCl was added (360 mL, 4 eq., based on 100% yield from Step 2) drop-wise over 20 minutes. Gas was evolved and the temperature rose slightly during addition. The suspension was heated to 35°C and after 4 hours a fluid slurry had formed with a 97% conversion being obtained (HPLC column: Symmetry Shield RP8, 3.5 µm, 50 mm; 230 nm - see Example 3 for more details). The mixture was cooled to 0 °C over 1 hour followed by a further period of 1 hour at 0°C. The material was then filtered and the crystals washed with a mixture of nBuOAc and EtOH (690 mL (2.3 vol.) and 210 mL (0.7 vol) respectively) followed by 900 mL of EtOAc (3 vol.) in order to wash out the nBuOAc. The product was dried under vacuum at 40 °C to give 237g of the dihydrochloride salt of Boc-Aze-CBA (0.94 mol, 87% yield over 2 steps, 98.9% purity by HPLC by analogy to Example 3).

The above deprotection can be repeated by using gaseous HCl in place of aqueous HCl.

The Pab(OMe) prepared according to Example 2 may then be coupled with Ph(3-Cl)(5-OCHF₂)-(*R*)CH(OH)C(O)-Aze-H to give Compound A.

### Example 3

The Pab(OMe) prepared according to Example 2 may also be coupled with Boc-Aze to give Boc-Aze-Pab(OMe). Alternatively, Boc-Aze-Pab(OMe) and Aze-Pab(OMe) may be prepared as follows:-

### Boc-Aze-Pab(OMe)

Boc-Aze-CBA (2.02g, 6.34mmol) was dissolved in EtOH (14mL) at ambient temperature. To the flask was added Triethylamine (3.1mL, 3.5 eq.), 30% MeONH₂ x HCl (aq) (3.5g, 2 eq.) and mercaptoacetic acid (0.45mL, 1 eq.). The solution was heated up to reflux and let to stand over night. After 22h at reflux, the reaction was at 97% conversion (HPLC). The mixture was then cooled to 40°C and acetone (0.95mL, 2 eq.) was added. The mixture was allowed to stand for 30 minutes to quench remaining MeONH₂. The amount of solvent was reduced by rotary evaporation under vacuum. The remaining residue was dissolved in n-BuOAc (16mL) och EtOH (6mL). After further evaporation water (15mL) was added and the phases was allowed to separate. The organic layer was washed twice with 2M K₂CO₃ solution( 2 x 20mL) and twice with water (2 x 20mL). The product solution was dried over Na₂SO₄ and evaporated to dryness. The residual was dissolved in *n*-BuOAc (11.2mL) and EtOH (3.5mL) and used in the following step.

### Aze-Pab(OMe) x 2HCl

To the above n-BuOAc/EtOH solution of Boc-Aze-Pab(OMe) from the previous step was added drop wise concentrated (12M) aqueous HCl (2.1mL, 4 eq.). The reaction mixture was heated up to 35°C. After approximately 3h the obtained slurry was sampled and analyzed to give <99% conversion by HPLC. The mixture was gradually cooled to 0°C and thereafter allowed to stand at 0° for at least 1h. The material was then filtered and the crystals were washed with a mixture of n-BuOAc (5.3mL) and EtOH (1.6mL) followed by EtOAc (6.9mL). The product was dried under vacuum at 40°C to give 1.50g of the dihydrochloride salt of Aze-Pab(OMe) (4.47mmol, 70.6% yield over 2 steps, 99.0% purity by HPLC).

This two step reaction has also been performed in MeOH in the first step resulting in a conversion of approximately 90% after 24h and 95% after 48h. The isolated yield after two steps were 67%.

### HPLC for conversion:

The reaction was monitored using Symmetry Shield RP8, 50 x 4.6mm, 3.5µm column. Mobile phase A (50mM NH₄H₂PO₄-buffert pH 3) and mobile phase B (CH₃CN/50mM NH₄H₂PO₄-buffert pH 3 70/30). Gradient 100% A for 10min, 0-100% B for 10 min, 100% B for 1 min, 0% A for 4 min. Flow 1.5mL/min at 230nm.

### HPLC for purify of Pab(OMe) x 2HCl

The isolated product was analyzed using ThermoHypersil Aquasil 100x4.6mm, 3µm. Mobile phase A (25mM NH₄H₂PO₄-buffert pH 3) and mobile phase B (CH₃CN/25mM NH₄H₂PO₄-buffert pH 3 80/20). Gradient 0-30% B for 15min, 30-100% B for 10min, 100% B for 5min, 0% B for 10min. Flow 1.0mL/min at 235nm.

### HPLC for purity of Aze-Pab(OMe) x 2HCl

The isolated product was analyzed using ThermoHypersil Aquasil 100x4.6mm, 3µm. Mobile phase A (25mM NH₄H₂PO₄-buffert pH 3) and mobile phase B (CH₃CN/25mM NH₄H₂PO₄-buffert pH 3 70/30). Gradient 0-30% B for 30min, 30-100% B for 10min, 100% B for 5min, 0% B for 10min. Flow 1.0mL/min at 235nm.

Aze-Pab(OMe) may then be coupled with 3-chloro-5-difluoromethoxy mandelic acid to give compound A. Aze-Pab(OMe) may also be coupled with other mandelic acids, so as to give compounds such as those described in WO 02/44145 A variety of different coupling conditions and coupling reagents (for example, reagents which facilitate the coupling by acting as dehydrating agents) are well known in the art and may be used to effect this reaction (preferably in high yield and with limited racemisation of the chiral mandelic acid). Suitable coupling reagents are EDC, TBTU, TCTU, HBTU, HCTU and PBOP, and these may be used in conjunction with hydroxybenzotriazole (HOBt). HOBt is preferably used as a tertiary amine salt, for example N-methylmorpholine.HOBt (NMM.HOBt), in aqueous solution in concentrations up to 50 wt.%. The use of aqueous solutions of HOBt tertiary amine salts (or aqueous mixtures of HOBt and a tertiary amine such as NMM) has advantages for handling such a reagent on large-scales.

Alternatively, the process of the invention may be applied to Ph(3-Cl)(5-OCHF₂)-(R)CH(OH)C(O)-Aze-CBA to give Compound A.

### Example 4-A

The process of the invention was applied to 3,5-difluorocyanobenzene to give 3,5-difluoro-N-methyl-benzamidoxime as follows...

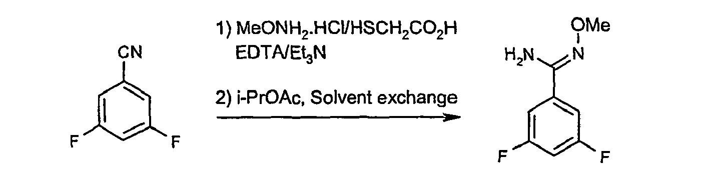

To a stirred solution of 3,5-difluorocyanobenzene (1 eq) in methanol (7 vol) under inert atmosphere was added EDTA (0.02% w/w calculated on the amount methoxylamine hydrochloride), triethylamine (4.5 eq), mercaptoacetic acid (1 eq) and methoxylamine hydrochloride (30% aq solution, 2 eq). Additions were carried out controlling the temperature below 25 °C, after which the reaction mixture was heated to reflux (65 °C) and left over night. The reaction mixture was then cooled to ≈ 35 °C and acetone (1.3 eq) for quenching of excess methoxylamine was added. After 60 mins the mixture was concentrated *in vacuo* to half original volume (ca 3.5 vol) and isopropylacetate was charged to original volume. The concentration procedure was repeated once and isopropylacetate was added again to original volume. From the resulting biphasic solution the aqueous phase was separated and was then re-extracted twice with isopropylacetate.

The combined organic phases were subsequently washed with Na₂CO₃ (aq. sat.) (2.5 vol) and NaCl (aq. sat.). After *in vacuo* removal of solvent the oily substance was applied on silica and eluted with ethylacetate. *In vacuo* removal of solvent afforded solid yellowish material in ≈ 97% yield with a purity of 98 area% by GC and LC.

### Example 4-B : HCl salt isolation

The HCl salt of 3,5-difluoro-N-methyl-benzamidoxime was isolated in high yield and very high purity as follows. The HCl salt facilitates both isolation and purification of the 3,5-difluoro-N-methyl-benzamidoxime product.

To a stirred solution of 3,5-difluorocyanobenzene (20.0 g, 144 mmol) in methanol (140 ml, 7 vol) under inert atmosphere was added EDTA (16.8 mg, 0.02% w/w calculated on the amount methoxylamine hydrochloride), triethylamine (90.2 mL, 647 mmol), mercaptoacetic acid (11.0 mL, 158 mmol) and methoxylamine hydrochloride (80,0 g, 288 mmol, 30% aq solution). Additions were carried out controlling the temperature below 25 °C, after which the reaction mixture was heated to reflux (65 °C) and left over night. The reaction mixture was then cooled below 40 °C and acetone (1.3 eq) for quenching excess methoxylamine was added. After 1h at 40 °C the mixture was concentrated *in vacuo* to approximately half original volume and isopropyl acetate was then charged to regain original volume. The concentration procedure was repeated once and isopropylacetate was added yet again to original volume. After removal of the organic layer the mixture was extracted twice more with isopropylacetate (2 x 40 mL).

The combined organic phases were subsequently washed with Na₂CO₃ (aq. sat.) (2 x 40 mL) and NaCl (aq. sat. 40mL) and then dried over MgSO₄. After filtration the solution was cooled to 10 °C. Upon slow addition of 4M HCl/1,4Dioxane (39,5 mL, 158 mmol) a solid precipitated out. After completed addition, the suspension was stirred for 20 min and then filtered off. Washing the residue with isopropyl acetate (40 mL) and subsequent drying *in vacuo* afforded 3,5-difluoro-N-methyl-benzamidoxime.HCl in 91 % yield (27.5 g, 131 mmol, 99.4 area% by LC).

The reaction was monitored on HPLC using Genesis AQ 100 x 4,6 mm, 4 µm column. Mobile phase A (CH₃CN/25 mM NaPO₄-buffert pH 3,0 5/95) and mobile phase B (CH₃CN/25 mM NaPO₄-buffert pH 3,0 60/40). Gradient 0-100 % B for 8 min, 100 % B for1 min, 100-0 % B for 0,1 min, 100 % A for 2,9 min. Flow 2,0 ml/min at 230/220 nm).

To a stirred suspension of 3,5-difluoro-N-methyl-benzamidoxime.HCl (20.0 g, 89.8 mmol) at r.t. in THF (140 mL) was slowly added Et₃N (18.8 mL, 135 mmol). The milky suspension was stirred for 5 h, after which the Et₃NHCl was filtered off. After washing the filter cake portion wise with THF (Σ 100 mL) the solution was either taken to the next step as it was, or the solvent was removed *in vacuo* to afford 3,5-difluoro-N-methyl-benzamidoxime as a pale yellow solid in 96% yield (16.1 g, 86.4 mmol, 98.2 area% by GC).

By analogy to Example 4, (2,6-diF)Pab(OMe) may be prepared from (2,6-diF)CBA (suitable protection, and later deprotection, of the amine functionality may be required, for example using a Boc-protecting group). The (2,6-diF)Pab(OMe) may then be coupled with Ph(3-Cl)(5-OCHF₂)-(*R*)CH(OH)C(O)-Aze-H to give Compound B.

### Example 5

The process of the invention was applied to Boc-Aze-(2,6-diF)CBA as follows.

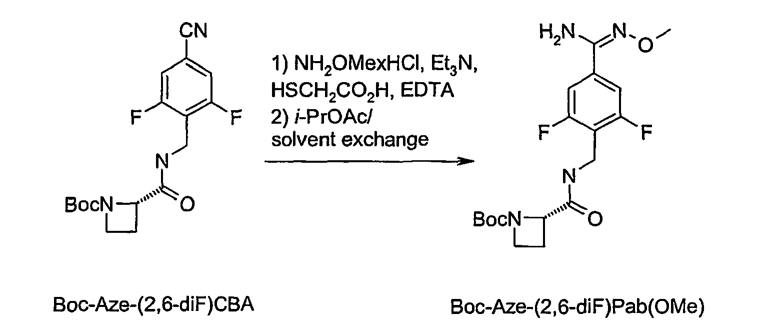

To a solution of Boc-Aze-(2,6-diF)CBA (1 eq) in methanol (7 vol,) under nitrogen atmosphere at room temperature was added EDTA (0.02% w/w calculated of the amount methoxylamine hydrochloride), triethylamine (4.5 eq) and mercaptoacetic acid (1 eq). To the mixture, methoxylamine hydrochloride (30% aq solution, 2 eq) was charged. The temperature raised +4 °C to 25 °C after complete addition. The reaction mixture was heated to 63 °C (oil bath 70 °C) and was left over night. HPLC analysis was carried out and showed > 99% conversion. The reaction mixture was cooled to < 50 °C and acetone for quenching of excess methoxylamine (1.2 eq) was added. After 30 min reaction (at 40-50 °C the mixture was concentrated *in vaccuo* to half original volume (ca 3.5 vol) and isopropylacetate was charged to original volume. The concentration procedure was repeated once and isopropylacetate was added again to original volume. The organic layer was washed with water (2 x 2 vol)) and water phase was separated off (pH ≈5). Organic layer was further washed with K₂CO₃ (aq sol, 2 M, 2x1 vol) and finally brine (20% w/w, 1 vol). The organic layer was used without any further purification after HPLC analysis, directly to next step, the debocylation procedure. An average yield was ca 90% (calculated with HPLC) with a HPLC purity of 93 - 96%.

The reaction was monitored on HPLC using Genesis AQ 100 x 4,6 mm, 4 µm column. Mobile phase A (CH₃CN/25 mM NaPO₄-buffert pH 3,0 5/95) and mobile phase B (CH₃CN/25 mM NaPO₄-buffert pH 3,0 60/40). Gradient 0-100 % B for 8 min, 100 % B for1 min, 100-0 % B for 0,1 min, 100 % A for 2,9 min. Flow 2,0 ml/min at 230/220 nm).

The Aze-(2,6-diF)Pab(OMe) may then be coupled with mandelic 3-chloro-5-difluoromethoxy mandelic acid to give compound B.

Alternatively, the process of the invention may be applied to Ph(3-Cl)(5-OCBF₂)-(*R*)CH(OH)C(O)-Aze-(2,6-diF)CBA to give Compound B.

Aze-(2,6-diF)Pab(OMe) may also be coupled with other mandelic acids, so as to give compounds such as those described in WO 02/44145

### Example 6

The process of the invention was evaluated on further substrates as follows.

### Example 6-A

4-Chlorobenzylnitrile was converted into the corresponding methoxyamidine by analogy to the method used in Example 4-B.

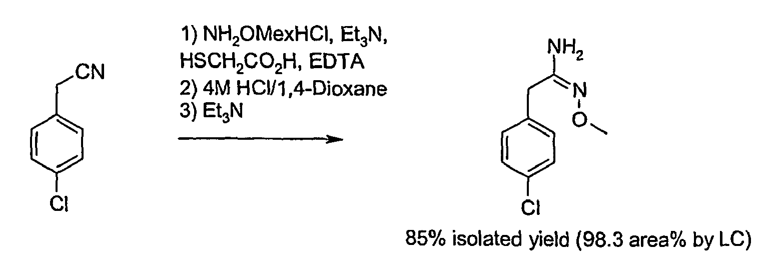

The product was characterized by ¹H /¹³C NMR and LCMS (ES+, m/z 199).

### Examples 6-B and 6-C

The following two substrates were reacted according by analogy to the method used in Example 4-A..

### Example 6-B

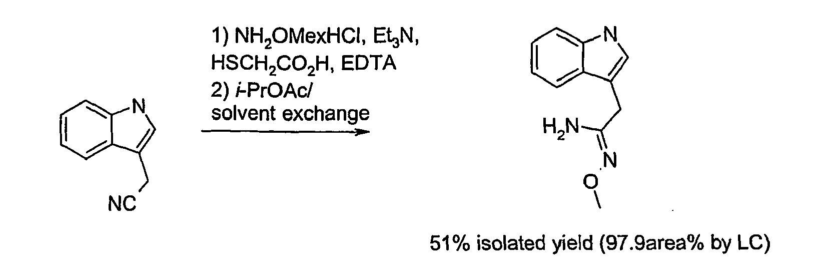

The product was characterized by ¹H /¹³C NMR and LCMS (ES+, m/z 204).

### Example 6-C

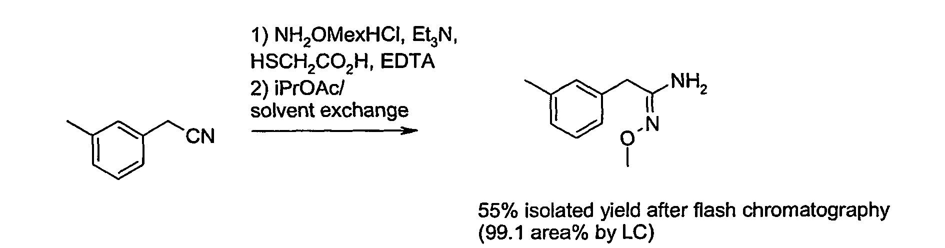

The product was characterized by ¹H /¹³C NMR and LCMS (ES+, m/z 179).

## Claims

1. A process for preparing a protected amidine group of formula (I) 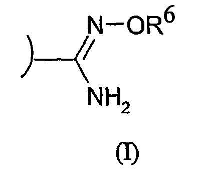 wherein R⁶ represents C₁₋₁₀ alkyl (optionally substituted by one or more substituents independently selected from halo, C₁₋₄ alkoxy, nitro, C₁₋₄ alkylamine and di-(C₁₋₄ alkyl)amine), aryl, C₁₋₃ alkylaryl or C₁₋₃ alkyloxyaryl, the alkyl parts of which latter two groups are optionally interrupted by one or more oxygen atoms, and the aryl parts of which latter two groups are optionally substituted by one or more substituents selected from halo, phenyl, methyl or methoxy, (which latter three groups are also optionally substituted by one or more halo substituents) which comprises
reacting a nitrile containing compound with an oxyamine of formula (II)
R⁶ONH₂ (II)
wherein R⁶ is as defined above for (I);
in the presence of a thio-keto activating agent of formula (III)
HS-R_{y}-C(O)-Z (III)
wherein Z is -(1-4C)alkyl, -OH, -O-(1-4C)alkyl, -SH, -S(1-4C)alkyl, -NH₂, -NH(1-4C)alkyl or -N[(1-4C)alkyl]₂;
R_{y} is (1-2C)alkyl, which is optionally substituted by up to three substituents independently selected from (1-4C)alkyl, halo, amino and acetylamino; or
Z and Ry are linked so as to form a 5- or 6-membered ring of formula (IV) 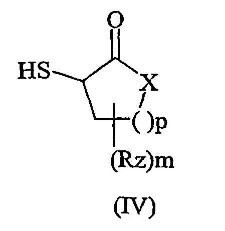 wherein
X is -CH₂-, -O-, NH- or -N(1-4C)alkyl; p is 1 or 2; m is 1 or 2 and
Rz is independently selected from H, (1-4C)alkyl, halo and amino.

2. A process according to claim 1 wherein R⁶ represents C₁₋₁₀ alkyl (preferably C₁₋₄ alkyl), aryl (preferably phenyl) or benzyl.

3. A process according to claim 1 or 2 wherein R⁶ represents methyl.

4. A process according to any of claims 1 to 3 wherein the thio-keto activating agent is used in quantitative amounts, i.e. equi-molar with the nitrile compound.

5. A process according to any of claims 1 to 4 wherein the thio-keto activating agent is mercapto-acetic acid.

6. A process according to any of claims 1 to 5 wherein the nitrile containing compound is a compound in which the nitrile group is attached to an aromatic or heteroaromatic ring.

7. A process according to any of claims 1 to 5 wherein the nitrile containing compound is a compound in which the nitrile group is attached to an alkyl chain or a cyclo-alkyl ring.

8. A process according to any of claims 1 to 6 wherein the nitrile containing compound is optionally protected para-cyano-benzylamine, 4-((*S*)-azetidine-2-carboxyaminomethyl)-cyanobenzene, *para*-cyano-(2,6-diF)benzylamine or 4-((*S*)-azetidine-2-carboxyaminomethyl)-(3,5-diF)cyanobenzene.

9. A process according to claim 8 wherein the nitrile containing compound is protected by a *tert*-butoxycarbonyl group.

10. The use of a thio-keto activating agent of formula (III)
HS-R_{y}-C(O)-Z (III)
wherein Z is -(1-4C)alkyl, -OH, -O-(1-4C)alkyl, -SH, -S(1-4C)alkyl, -NH₂, -NH(1-4C)alkyl or -N[(1-4C)alkyl]₂;
R_{y} is (1-2C)alkyl, which is optionally substituted by up to three substituents independently selected from (1-4C)alkyl, halo, amino and acetylamino; or
Z and Ry are linked so as to form a 5- or 6-membered ring of formula (IV) 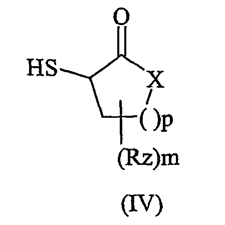 wherein
X is -CH₂-, -O-, NH- or -N(1-4C)alkyl; p is 1 or 2; m is 1 or 2 and
Rz is independently selected from H, (1-4C)alkyl, halo and amino in a process according to any of claims 1 to 9.

## Patentansprüche

1. Verfahren zur Herstellung einer geschützten Amidingruppe der Formel (I) 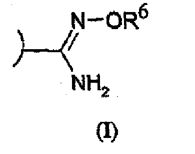 worin R⁶ für C₁₋₁₀-Alkyl (gegebenenfalls substituiert durch einen oder mehrere unabhängig voneinander unter Halogen, C₁₋₄-Alkoxy, Nitro, C₁₋₄-Alkylamino und Di(C₁₋₄-alkyl)amino ausgewählte Substituenten), Aryl, C₁₋₃-Alkylaryl oder C₁₋₃-Alkyloxyaryl steht, die Alkylteile der letztgenannten zwei Gruppen gegebenenfalls durch ein oder mehrere Sauerstoffatome unterbrochen sind und die Arylteile der letztgenannten zwei Gruppen gegebenenfalls durch einen oder mehrere unter Halogen, Phenyl, Methyl oder Methoxy ausgewählte Substituenten substituiert sind (wobei die letztgenannten drei Gruppen außerdem gegebenenfalls durch einen oder mehrere Halogensubstituenten substituiert sind), bei dem man
eine nitrilgruppenhaltige Verbindung
in Gegenwart eines Thio-Keto-Aktivierungsmittels der Formel (III)
HS-R_{y}-C(O)-Z (III)
worin Z für -C₁₋₄-Alkyl, -OH, -O-C₁₋₄-Alkyl, -SH, -S-C₁₋₄-Alkyl, -NH₂, -NH-C₁₋₄-Alkyl oder -N (C₁₋₄-Alkyl)₂ steht;
R_{y} für C₁₋₂-Alkyl, das gegebenenfalls durch bis zu drei unabhängig voneinander unter C₁₋₄-Alkyl, Halogen, Amino und Acetylamino ausgewählte Substituenten substituiert ist, steht; oder Z und R_{y} so verknüpft sind, daß sie einen 5- oder 6-gliedrigen Ring der Formel (IV) 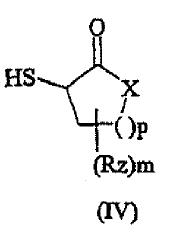 bilden, worin
X für -CH₂-, -O-, -NH- oder -N-C₁₋₄-Alkyl steht; p für 1 oder 2 steht; m für 1 oder 2 steht und Rz unabhängig unter H, C₁₋₄-Alkyl, Halogen oder Amino ausgewählt ist;
mit einem Oxyamin der Formel (II)
R⁶ONH₂ (II)
worin R⁶ die oben für (I) angegebene Bedeutung besitzt;
umsetzt.

2. Verfahren nach Anspruch 1, bei dem R⁶ für C₁₋₁₀-Alkyl (vorzugsweise C₁₋₄-Alkyl), Aryl (vorzugsweise Phenyl) oder Benzyl steht.

3. Verfahren nach Anspruch 1 oder 2, bei dem R⁶ für Methyl steht.

4. Verfahren nach einem der Ansprüche 1 bis 3, bei dem man das Thio-Keto-Aktivierungsmittel in quantitativen Mengen, d.h. äquimolar mit der Nitrilverbindung, verwendet.

5. Verfahren nach einem der Ansprüche 1 bis 4, bei dem es sich bei dem Thio-Keto-Aktivierungsmittel um Mercaptoessigsäure handelt.

6. Verfahren nach einem der Ansprüche 1 bis 5, bei dem es sich bei der nitrilgruppenhaltigen Verbindung um eine Verbindung handelt, in der die Nitrilgruppe an einen aromatischen oder heteroaromatischen Ring gebunden ist.

7. Verfahren nach einem der Ansprüche 1 bis 5, bei dem es sich bei der nitrilgruppenhaltigen Verbindung um eine Verbindung handelt, in der die Nitrilgruppe an eine Alkylkette oder einen Cycloalkylring gebunden ist.

8. Verfahren nach einem der Ansprüche 1 bis 6, bei dem es sich bei der nitrilgruppenhaltigen Verbindung um gegebenenfalls geschütztes *para*-Cyanobenzylamin, 4-((*S*)-Azetidin-2-carboxyaminomethyl)-cyanobenzol, *para*-Cyano-(2,6-diF)benzylamin oder 4-((*S*)-Azetidin-2-carboxyaminomethyl)-(3,5-diF)-cyanobenzol handelt.

9. Verfahren nach Anspruch 8, bei dem die nitrilgruppenhaltige Verbindung durch eine tert-Butoxycarbonylgruppe geschützt ist.

10. Verwendung eines Thio-Keto-Aktivierungsmittels der Formel (III)
HS-R_{y}-C(O)-Z (III)
worin Z für -C₁₋₄-Alkyl, -OH, -O-C₁₋₄-Alkyl, -SH, -S-C₁₋₄-Alkyl, -NH₂, -NH-C₁₋₄-Alkyl oder -N(C₁₋₄-Alkyl)₂ steht;
R_{y} für C₁₋₂-Alkyl, das gegebenenfalls durch bis zu drei unabhängig voneinander unter C₁₋₄-Alkyl, Halogen, Amino und Acetylamino ausgewählte Substituenten substituiert ist, steht; oder
Z und R_{y} so verknüpft sind, daß sie einen 5- oder 6-gliedrigen Ring der Formel (IV) 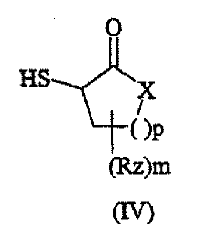 bilden, worin
X für -CH₂-, -O-, -NH- oder -N-C₁₋₄-Alkyl steht; p für 1 oder 2 steht; m für 1 oder 2 steht und Rz unabhängig unter H, C₁₋₄-Alkyl, Halogen oder Amino ausgewählt ist;
bei einem Verfahren nach einem der Ansprüche 1 bis 9.

## Revendications

1. Procédé de préparation d'un groupe amidine protégé de la formule (I) 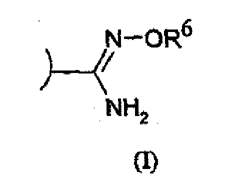 dans laquelle R⁶ représente un groupe alkyle de C₁ à C₁₀ (éventuellement substitué par un ou plusieurs substituants indépendamment choisis parmi les groupes halo, alcoxy de C₁ à C₄, nitro, alkylaryle de C₁ à C₄ et di-(alkyle de C₁ à C₄) amine), aryle, alkylaryle de C₁ à C₃ ou alkyloxyaryle de C₁ à C₃, dont les parties alkyle des deux derniers groupes sont éventuellement interrompus par un ou plusieurs atomes d'oxygène, et dont les parties aryle des deux derniers groupes sont éventuellement substitués par un ou plusieurs substituants choisis parmi les groupes halo, phényle, méthyle ou méthoxy (ces trois derniers groupes étant également éventuellement substitués par un ou plusieurs substituants halo)
lequel comprend la réaction d'un nitrile qui contient un composé avec une oxyamine de la formule (II)
R⁶ONH₂ (II)
dans laquelle R⁶ est tel que défini ci-dessus pour (I) ;
en la présence d'un agent d'activation thio-céto de la formule (III)
HS-R_{y}-C(O)-Z (III)
dans laquelle Z représente un groupe -alkyle (C₁ à C₄), -OH, -O-alkyle (C₁ à C₄), -SH, -S-alkyle (C₁ à C₄), -NH₂, -NH-alkyle (C₁ à C₄) ou -N [alkyle (C₁ à C₄)]₂ ;
R_{y} représente un groupe alkyle (C₁ à C₂), lequel est éventuellement substitué par jusqu'à trois substituants indépendamment choisis parmi les groupes alkyle (C₁ à C₄), halo, amino et acétylamino ; ou
Z et R_{y} sont liés de sorte à former un cycle à cinq ou six chaînons de la formule (IV) 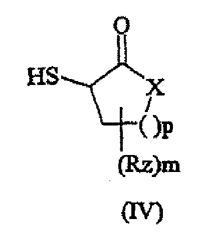 dans laquelle
X représente un groupe -CH₂-, -O-, -NH- ou -N-alkyle (C₁ à C₄) ; p vaut 1 ou 2 ; m vaut 1 ou 2 et
R_{z} est indépendamment choisi parmi un atome H, les groupes alkyle (C₁ à C₄), halo et amino.

2. Procédé selon la revendication 1, dans lequel R⁶ représente un groupe alkyle de C₁ à C₁₀ (de préférence un groupe alkyle de C₁ à C₄), aryle (de préférence un groupe phényle) ou benzyle.

3. Procédé selon la revendication 1 ou la revendication 2, dans lequel R⁶ représente un groupe méthyle.

4. Procédé selon l'une quelconque des revendications 1 à 3, dans lequel l'agent d'activation thio-céto est utilisé en des quantités quantitatives, c.-à-d., équimolaires avec le composé contenant un groupe nitrile.

5. Procédé selon l'une quelconque des revendications 1 à 4, dans lequel l'agent d'activation thio-céto est l'acide mercapto-acétique.

6. Procédé selon l'une quelconque des revendications 1 à 5, dans lequel le composé contenant un groupe nitrile est un composé dans lequel le groupe nitrile est attaché à un noyau aromatique ou hétéroaromatique.

7. Procédé selon l'une quelconque des revendications 1 à 5, dans lequel le composé contenant un groupe nitrile est un composé dans lequel le groupe nitrile est attaché à une chaîne alkyle ou à un noyau cycloalkyle.

8. Procédé selon l'une quelconque des revendications 1 à 6, dans lequel le composé contenant un groupe nitrile est éventuellement protégé par un groupe para-cyano-benzylamine, 4-((S)-azétidine-2-carboxyaminométhyl)-cyanobenzène, para-cyano-(2,6-diF)benzylamine ou 4-((S)-azétidine-2-carboxyaminométhyl)-(3,5-diF)cyanobenzène.

9. Procédé selon la revendication 8, dans lequel le composé contenant un groupe nitrile est protégé par un groupe tert-butoxycarbonyle.

10. Utilisation d'un agent d'activation thio-céto de la formule (III)
HS-R_{y}-C(O)-Z (III)
dans laquelle Z représente un groupe -alkyle (C₁ à C₄), -OH, -O-alkyle (C₁ à C₄), -SH, -S-alkyle (C₁ à C₄), -NH₂, -NH-alkyle (C₁ à C₄) ou -N [alkyle (C₁ à C₄)]₂ ;
R_{y} représente un groupe alkyle (C₁ à C₂), lequel est éventuellement substitué par jusqu'à trois substituants indépendamment choisis parmi les groupes alkyle (C₁ à C₄), halo, amino et acétylamino ; ou
Z et R_{y} sont liés de sorte à former un cycle à cinq ou six chaînons de la formule (IV) 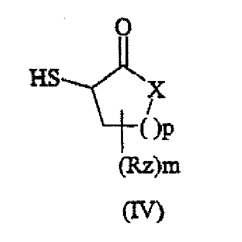 dans laquelle
X représente un groupe -CH₂-, -O-, -NH- ou -N-alkyle (C₁ à C₄) ; p vaut 1 ou 2 ; m vaut 1 ou 2 et
R_{z} est indépendamment choisi parmi un atome H, les groupes alkyle (C₁ à C₄), halo et amino dans un procédé selon l'une quelconque des revendications 1 à 9.
